# EUROPÄISCHE PATENTANMELDUNG

(11) **EP 1 924 123 A2**
(43) Veröffentlichungstag der Anmeldung: **21.05.2008**
(21) Anmeldenummer: 07021860.7
(22) Anmeldetag: 10.11.2007
(51) Int. Cl.: H04R 25/00

(54) **Hörgerät**

(30) Priorität: 17.11.2006 DE 202006017662 U
(71) Anmelder: Bagus GmbH & Co. KG, 45276 Essen (DE)
(72) Erfinder: Bagus, Reinhold, 45276 Essen (DE)
(74) Vertreter: Nunnenkamp, Jörg

(57) **Zusammenfassung**

Gegenstand der vorliegenden Erfindung ist ein Hörgerät, mit zumindest einer externen Signaleinheit (11, 11', 12, 13, 14', 14", 15), wobei wenigstens ein implantierbares Befestigungselement (17) für die mit einem Gegenbefestigungselement (18) ausgerüstete Signaleinheit (11, 11', 12, 13, 14, 14', 14", 15) vorgesehen ist. Erfindungsgemäß ist ein die Signaleinheit (11, 11', 12, 13, 14', 14", 15) aufnehmendes Gehäuse (15) aus einer die Signaleinheit (11, 11', 12, 13, 14', 14", 15) umschließenden aushärtenden Kunststoffmasse gefertigt.

## Beschreibung

Die Erfindung betrifft ein Hörgerät, mit zumindest einer externen Signaleinheit, wobei wenigstens ein implantierbares Befestigungselement für die mit einem Gegenbefestigungselement ausgerüstete Signaleinheit vorgesehen ist.

Ein solches Hörgerät wird in der DE 20 2004 006 117 U1 vorgestellt. Hier geht es um ein Knochenleitungshörgerät mit Magnetankopplung. Dieses verfügt über eine externe Signaleinheit in Gestalt eines Vibrators. Die Signalübertragung erfolgt von dem Vibrator in einen zugehörigen Magneten. Der vorerwähnte Magnet wird durch einen zweiten, operativ implantierten Magneten fixiert.

Der vom Vibrator erzeugte Schall wird als Festkörperschall durch die Haut und subkutanes Gewebe in den implantierten Magneten und von dort aus weiter in einen unmittelbar anliegenden Knochen übertragen.

Darüber hinaus wird ein Hörgerät in der DE 20 2005 009 021 U1 beschrieben. Dieses ist als Knochenhörgerät ausgebildet, bei welchem zusätzlich zu der dortigen Signaleinheit bzw. einer Signalbearbeitungs- und Verstärkungseinheit ein elektromechanischer Wandler in Gestalt eines Vibrators realisiert ist. Der elektromechanische Wandler mag unmittelbar am Schädelknochen bzw. dem so genannten Mastoid eines Benutzers anliegen. Auf diese Weise werden seine Vibrationen per Körperschall zum an sich funktionsfähigen Innenohr des fraglichen Benutzers weitergeleitet. - Das bekannte Hörgerät ist lösbar an einem Tragbügelbogen befestigt. Das hat sich grundsätzlich bewährt.

Darüber hinaus kennt man durch beispielsweise die DE 39 18 329 A1 ebenfalls gattungsgemäße Hörgeräte, die allerdings zur elektrischen Reizung des (menschlichen) Innenohres eingesetzt werden. Dazu verfügen diese Hörgeräte über eine implantierbare Reizelektrodenanordnung. Die externe Signaleinheit ist in diesem Fall induktiv mit der Reizelektrodenanordnung gekoppelt.

Dabei wird üblicherweise so vorgegangen, dass akustische Signale mit Hilfe der Signaleinheit in elektrische Signale umgewandelt werden, die ihrerseits die Reizelektrodenanordnung beaufschlagen. Die Reizelektrodenanordnung mag eine kugelförmige aktive Elektrode sowie eine neutrale Elektrode aufweisen. Die aktive Elektrode kann beispielsweise in das menschliche Innenohr eingeführt werden und hier den dort vorhandenen Hörnerv oder jedenfalls noch stimulierbare Reste desselben elektrisch reizen. Dadurch wird ein Höreindruck hervorgerufen, mit dessen Hilfe Gehörlose und Spätertaubte nach einer gewissen Trainingszeit erstmals oder wieder hören können.

Ähnlich geht die US-PS 4,532,930 vor. Hier wird die Signaleinheit nicht - wie bei der DE 39 18 329 A1 - als hinter dem Ohr zu tragendes Gehäuse ausgeführt, sondern ist in einer mitzuführenden Tasche untergebracht. Von dieser Tasche reicht ein Verbindungskabel zu einer Sendeantenne, die im Bereich des betreffenden Ohres getragen wird.

Die beschriebenen Vorgehensweisen können hinsichtlich des Tragekomforts nicht wirklich überzeugen und sind deshalb verbesserungsfähig. Hier setzt die Erfindung ein.

Der Erfindung liegt das technische Problem zugrunde, ein Hörgerät der eingangs beschriebenen Ausführungsform so weiterzuentwickeln, dass der Tragekomfort deutlich verbessert ist.

Zur Lösung dieser technischen Problemstellung ist ein gattungsgemäßes Hörgerät im Rahmen der Erfindung dadurch gekennzeichnet, dass ein die Signaleinheit aufnehmendes Gehäuse bzw. Signalgehäuse aus einer die Signaleinheit umschließenden aushärtenden Kunststoffmasse gefertigt ist.

In diesem Fall wird die Signaleinheit bzw. werden die einzelnen Elemente der Signaleinheit von der fraglichen Kunststoffmasse vorteilhaft durch einen Umspritzvorgang umschlossen, welcher zugleich das Signalgehäuse als solches definiert. Dadurch wird nicht nur die Signaleinheit hermetisch vor Umwelteinflüssen geschützt, sondern kann beispielsweise das Signalgehäuse problemlos an Farbwünsche des Bedieners angepasst werden.

In jedem Fall wird die Signaleinheit durch das Zusammenspiel von Befestigungselement und Gegenbefestigungselement am Kopf eines Bedieners gehalten, und zwar vorzugsweise lösbar. Dabei handelt es sich bei dem Befestigungselement und dem Gegenbefestigungselement um zwei Magnete. Alternativ oder zusätzlich kann aber auch auf einen Magneten und ein magnetisierbares Element, beispielsweise eine Stahlscheibe, ein Stahlplättchen oder dergleichen zurückgegriffen werden. Dadurch ist der Tragekomfort enorm verbessert.

Denn nun ist es nicht mehr erforderlich, das Gehäuse bzw. Signalgehäuse zur Aufnahme der Signaleinheit hinter dem Ohr eines Anwenders platzieren zu müssen. Das heißt, das Außenohr als gleichsam Träger des Signalgehäuses wird erfindungsgemäß nicht mehr benötigt (vgl. DE 39 18 329 A1). Außerdem entfallen komplizierte Taschenlösungen, wie sie in der US-PS 4,532,930 vorgestellt werden. Vielmehr wird die Signaleinheit praktisch frei am Kopf eines Bedieners, vorteilhaft hinter dessen Ohr, (lösbar) gehalten. Zusätzliche Haltemaßnahmen, wie das Außenohr, ggf. ein Brillenbügel oder ein Tragebügel sind nicht (mehr) erforderlich (vgl. DE 20 2005 009 021 U1).

Immer lässt sich die Signaleinheit bzw. das die Signaleinheit aufnehmende Gehäuse respektive Signalgehäuse problemlos von einer Anlagefläche am Kopf eines Bedieners entfernen und hieran wieder anlegen, weil hierzu lediglich die von dem Befestigungselement und Gegenbefestigungselement aufgebrachten Magnethaltekräfte überwunden bzw. aufgebracht werden müssen. In diesem Zusammenhang greift die Erfindung vorteilhaft auf (Neodym-) Permanent-Magnete zurück, die äußerst klein bauen und problemlos die erforderlichen Haltekräfte aufbauen.

Tatsächlich können die Signaleinheit und mit ihr das Signalgehäuse äußerst kompakt gestaltet werden, weil durch die zunehmende Miniaturisierung im Elektronikbereich die Einzelkomponenten der Signaleinheit kaum Platz benötigen. Beobachtet werden an dieser Stelle Volumina für das Signalgehäuse, die im Bereich von ca. 1 cm³ oder auch darunter liegen, folglich von einem Anwender nicht als störend empfunden werden. Hinzu kommt, dass das Gewicht im Bereich von wenigen Gramm (< 0,1 N) angesiedelt ist, so dass die eingesetzten Magnete problemlos die zur Fixierung der Signaleinheit bzw. des Signalgehäuses erforderlichen Haltekräfte aufbringen können.

Eine besonders vorteilhafte Anbringung der Signaleinheit ist dann realisiert, wenn zwei oder mehr Paare des Befestigungselementes/Gegenbefestigungselementes realisiert sind. Beispielsweise hat es sich bewährt, wenn die entsprechenden Paare jeweils randseitig des Signalgehäuses zur Aufnahme der Signaleinheit platziert werden. Das ist problemlos möglich, zumal das Signalgehäuse als kapselartiges Kunststoffgehäuse ausgebildet ist und eine abgerundete Quaderform besitzt.

Eine weitere Anpassung ist dergestalt möglich, dass das Befestigungselement und/oder das Gegenfestigungselement verstellbar, insbesondere Höhenverstellbar, ausgebildet sind. Hierdurch lässt sich der Abstand einer Grundplatte des Signalgehäuses von der Anlagefläche am Kopf des Bedieners verändern. Diese Maßnahme trägt ergänzend zur Erhöhung des Tragekomforts bei. Dabei mag die Grundplatte insgesamt austauschbar und als Abdruckplatte ausgebildet sein. Dadurch lässt sich von der Anlagefläche am Kopf des Bedieners ein Abdruck nehmen und auf diese Weise die Grundplatte im Signalgehäuse an den jeweiligen Bediener anpassen.

Die Signaleinheit verfügt - wie bereits ausgeführt - über mehrere Elemente. Hierzu gehören regelmäßig wenigstens ein Mikrophon, ein Signalprozessor und eine Sendespulenanordnung. Alternativ oder zusätzlich mögen auch eine Signalbearbeitungs- und Verstärkungseinheit sowie ein elektromechanischer Wandler zu der Signaleinheit gehören. Mit Hilfe des Signalprozessors bzw. der Signalbearbeitungs- und Verstärkungseinheit werden die vom Mikrophon aufgenommenen akustischen Signale in elektrische Signale umgewandelt.

Diese elektrischen Signale steuern im Falle eines so genannten Knochenhörgerätes den elektromechanischen Wandler an, welcher auf diese Weise in die gewünschten Vibrationen versetzt wird. Bei einem Hörgerät zur elektrischen Reizung des menschlichen Innenohres werden die fraglichen elektrischen Signale an die Sendespulenanordnung weitergegeben, die mit einer implantierten Empfangsspulenanordnung induktiv gekoppelt ist. Die Empfangsspulenanordnung ist mit der Reizelektrodenanordnung elektrisch verbunden. Zu der Empfangsspulenanordnung mag ein optional vorgesehener Empfangsprozessor gehören, von dem die eine oder die mehreren Elektroden der Reizelektrodenanordnung ausgehen, um das Innenohr - wie beschrieben - elektrisch zu reizen.

In beiden Fällen können die elektrischen Signale in Digitalsignale umgewandelt werden bzw. liegen als solche vor und stellen daher rechnerisch zu verarbeitende Digitaldaten dar. Dabei können gebräuchliche Algorithmen zum Einsatz kommen, die die akustischen Signale für die anschließende Ansteuerung des elektromechanischen Wandlers bzw. die anschließende drahtlose Kopplung mit der Reizelektrodenanordnung bedienerabhängig aufbereiten. Darunter wird im Rahmen der Erfindung eine Anpassung verstanden, die je nach Anatomie des Innenohres des Bedieners bzw. Patienten eine individuelle Signalaufbereitung im Signalprozessor bzw. in der Signalbearbeitungs- und Verstärkungseinheit vornimmt. Meistens wird die fragliche Anpassung als bedienerabhängiges Softwareprogramm im Signalprozessor bzw. der Signalbearbeitungs- und Verstärkungseinheit hinterlegt.

Dadurch, dass die Signaleinheit und mit ihr der Signalprozessor bzw. die Signalbearbeitungs- und Verstärkungseinheit extern angeordnet sind, lässt sich die den Signalprozessor bzw. die Signalbearbeitungs- und Verstärkungseinheit steuernde Software problemlos aktualisieren. Ebenso kann eine Energieversorgungseinheit, beispielsweise Batterie, turnusgemäß ausgetauscht werden, die ebenfalls im Signalgehäuse aufgenommen wird. Hierzu verfügt das als kapselartiges Kunststoffgehäuse ausgebildete Signalgehäuse über eine verschließbare Öffnung zur Aufnahme der betreffenden Energieversorgungseinheit.

Die Signaleinheit mag vorteilhaft an einem hinter dem Außenohr liegenden Knochenbereich, dem sog. Mastoid, angeordnet werden. Tatsächlich eignet sich dieser Knochenbereich bzw. das Mastoid besonders vorteilhaft dazu, die Befestigungselemente zu implantieren. Außerdem nimmt das Mastoid im Falle des Hörgerätes zur elektrischen Reizung die dazu erforderliche Reizelektrodenanordnung auf. Bei einem Knochenhörgerät dient das Mastoid zur Übertragung der Vibrationen des elektromechanischen Wandlers per Körperschall an das Innenohr.

Insgesamt wird ein Hörgerät zur Verfügung gestellt, welches über einen bisher nicht gekannten Tragekomfort verfügt. Denn die obligatorische externe Signaleinheit lässt sich problemlos von der Anlagefläche am Kopf des Bedieners bzw. Patienten entfernen. Auch eine Wiederanbringung ist unschwer möglich, weil das Zusammenspiel von implantiertem Befestigungselement und externem Gegenbefestigungselement an oder in der Signaleinheit lediglich auf magnetischen Haltekräften basiert. Diese können unschwer aufgehoben und wieder hergestellt werden. Hierin sind die wesentlichen Vorteile zu sehen.

Im Folgenden wird die Erfindung anhand einer lediglich ein Ausführungsbeispiel darstellenden Zeichnung näher erläutert, es zeigen:
- Fig. 1: das erfindungsgemäße Hörgerät in der Ausführungsform als Knochenhörgerät schematisch,
- Fig. 2: das Hörgerät nach der Erfindung in der Ausgestaltung als Hörgerät zur elektrischen Innenohrreizung und
- Fig. 3: das Hörgerät nach den Fig. 1 und 2 im Detail.

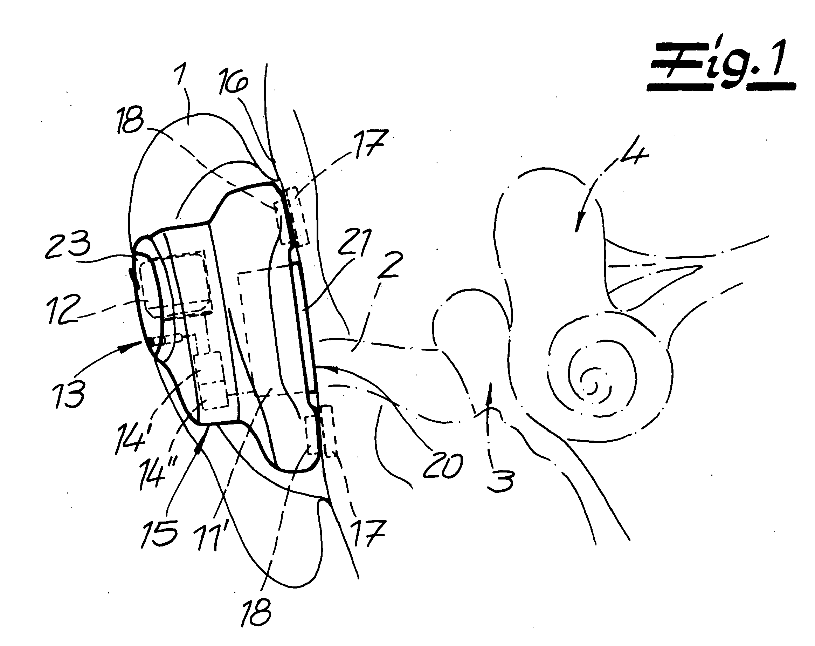

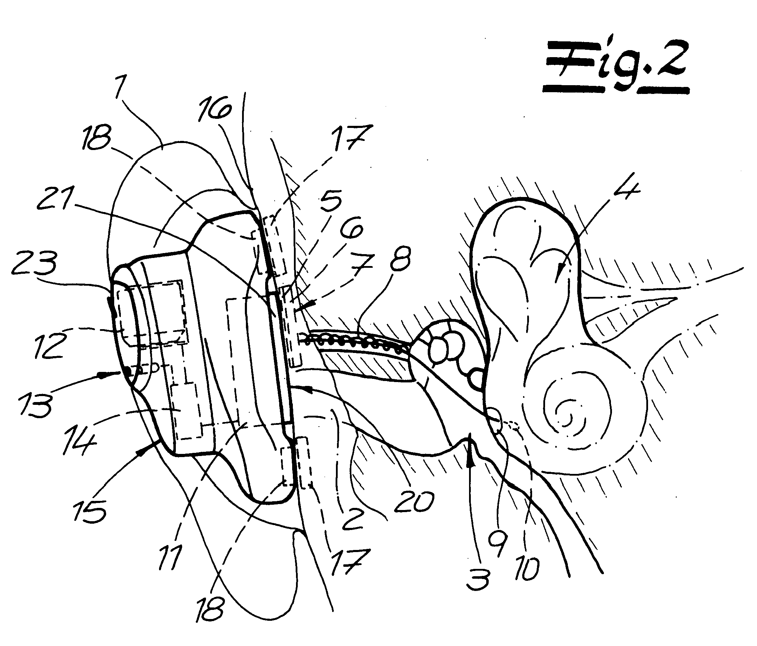

In der Fig. 1 erkennt man ein menschliches Ohr teilweise im Schnitt. Dieses unterteilt sich in das äußere Ohr mit Ohrmuschel 1 und dem äußeren Gehörgang 2, das Mittelohr 3 und schließlich das Innenohr 4, welches für die nachfolgenden Betrachtungen von besonderer Bedeutung ist. Denn im Rahmen der Erfindung wird einerseits ein Knochenhörgerät nach Fig. 1 verfolgt, welches mit Hilfe eines elektromechanisches Wandlers 11' in Gestalt eines Vibrators am Mastoid des Benutzers anliegt und auf diese Weise Vibrationen des elektromechanischen Wandlers 11' per Körperschall an das an sich funktionsfähige Innenohr 4 überträgt. Andererseits dient das Hörgerät zur elektrischen Reizung des vorerwähnten Innenohres 4. Hierzu verfügt das fragliche Hörgerät über eine implantierbare Reizelektrodenanordnung 5, 6, 7, 8, 9, 10, die in der zugehörigen Fig. 2 dargestellt ist.

In beiden Fällen der verschiedenen Ausgestaltungen nach den Fig. 1 und 2 erkennt man eine Signaleinheit 11, 11', 12, 13, 14, 14' und 14" sowie 15, die zum einen den elektromechanischen Wandler 11' beaufschlagt und zum anderen induktiv mit der vorerwähnten Reizelektrodenanordnung 5, 6, 7, 8, 9, 10 gekoppelt ist.

Die Reizelektrodenanordnung 5, 6, 7, 8, 9, 10 setzt sich im Detail aus einer Empfangsspulenanordnung 5 sowie einem die Signale der Empfangsspulenanordnung 5 verarbeitenden Empfangsprozessor 6 zusammen. Empfangsspulenanordnung 5 und Empfangsprozessor 6 werden gemeinsam in einem biokompatiblen und hermetisch verschlossenen Gehäuse 7 aufgenommen, welches unter die Kopfhaut 16 eines Patienten bzw. Bedieners implantiert wird, und zwar im Knochenbereich hinter dem Außenohr bzw. der Ohrmuschel 1 (Mastoid). Dieser Anbringungsort empfiehlt sich aufgrund der geringen Anzahl an Nervensträngen in diesem Bereich.

Die mit Hilfe der Empfangsspulenanordnung 5 von einer Sendespulenanordnung 11 der Signaleinheit 11, 11', 12, 13, 14, 14', 14", 15 empfangenen hochfrequenten elektrischen Signale werden im Empfangsprozessor 6 aufbereitet und an eine Elektrode 10 im Innenohr 4 weitergeleitet, die den dortigen Hörnerv oder dessen Überreste elektrisch stimuliert. Zu diesem Zweck wird die fragliche aktive Elektrode 10 von einer neutralen Elektrode 8 umwendelt, welche eine Zuleitung der aktiven Elektrode 10 auf einer gewissen Strecke umhüllt. Die aktive Elektrode 10 wird durch ein Fenster 9 in das Innenohr 4 und in den Bereich des Hörnerves geführt.

Die externe Signaleinheit 11, 11', 12, 13, 14, 14', 14", 15 setzt sich aus der bereits angesprochenen Sendespulenanordnung 11, einer Energieversorgungseinrichtung 12, vorliegend einer Batterie, einem Mikrophon 13, einem Signalprozessor 14 sowie schließlich einem Gehäuse bzw. Signalgehäuse 15 im Rahmen des Ausführungsbeispiels nach Fig. 2 zusammen, welches die gesamte dortige Signaleinheit 11, 12, 13, 14, 15 bzw. die vorerwähnten Elemente 11, 12, 13, 14 umschließt.

Bei der Variante nach Fig. 1 umfasst die Signaleinheit 11', 12, 13, 14', 14", 15 den bereits angesprochenen elektromechanischen Wandler 11', die Energieversorgungseinrichtung 12, das Mikrophon 13, eine Signalbearbeitungseinheit 14' sowie eine Verstärkungseinheit 14" und schließlich das Gehäuse 15. Die Signalbearbeitungseinheit 14' beeinflusst das vom Mikrophon 13 aufgenommene Signalspektrum des akustischen Signals in der Weise, dass tendenziell hohe Frequenzen mehr als tiefe Frequenzen verstärkt werden. Dadurch wird der anschließenden Körperschallübertragung mit Hilfe des elektromechanischen Wandlers 11' Rechnung getragen. Nach dem das betreffende akustische Signal in der beschriebenen Weise aufbereitet worden ist, wird es in der Verstärkungseinheit 14" verstärkt und an den fraglichen elektromechanischen Wandler 11' übertragen, um diesen zu Vibrationen anzuregen (vgl. Fig. 1). Bei dieser Variante umschließt das Gehäuse 15 die Elemente 11', 12, 13, 14' und 14".

Dadurch, dass der elektromechanische Wandler 11' unmittelbar bzw. unter Zwischenschaltung einer Grundplatte 21 am Mastoid des Benutzers anliegt, werden seine Vibrationen per Körperschall zum funktionsfähigen Innenohr des Benutzers weitergeleitet. Weil diese Körperschallübertragung niedrige gegenüber hohen Frequenzen bevorzugt, findet ein Ausgleich der zuvor vorgenommenen unterschiedlichen Verstärkung mit dem Ergebnis statt, dass im Idealfall das ursprüngliche akustische Spektrum vom Innenohr 4 aufgenommen und im Gehirn umgesetzt wird.

In beiden Fällen ist das Gehäuse bzw. Signalgehäuse 15 aus einer aushärtenden Kunststoffmasse gefertigt und wird dadurch hergestellt, dass die einzelnen Elemente 11, 11', 13, 14 und 14', 14" - bis auf die Energieversorgungseinrichtung 12 - zusammengenommen von der aushärtenden Kunststoffmasse umspritzt und hierin gleichzeitig unter Bildung des Gehäuses 15 eingebettet werden. Dadurch kann das Gehäuse bzw. Signalgehäuse 15 beispielsweise durchsichtig oder auch farbig nach den Wünschen des Bedieners gestaltet werden.

Für die Erfindung von besonderer Bedeutung ist nun, dass neben der implantierten bzw. implantierbaren Reizelektrodenanordnung 5, 6, 7, 8, 9, 10 im Rahmen der Fig. 2 bzw. unabhängig hiervon bei der Ausführungsform nach Fig. 1 (zusätzlich) wenigstens ein Befestigungselement 17 implantiert wird. Tatsächlich sind zwei implantierte bzw. implantierbare Befestigungselemente 17 vorgesehen, die in unmittelbarer Nachbarschaft zum Gehäuse 7 für die Aufnahme der Empfangsspulenanordnung 5 sowie des Empfangsprozessors 6 unter der Kopfhaut 16 angeordnet sind, und zwar wiederum im Mastoid (vgl. Fig. 2). Bei der Variante nach Fig. 1 findet die Anhängung der Befestigungselemente 17 ebenfalls in dem besagten Knochenbereich statt, allerdings örtlich praktisch frei wählbar.

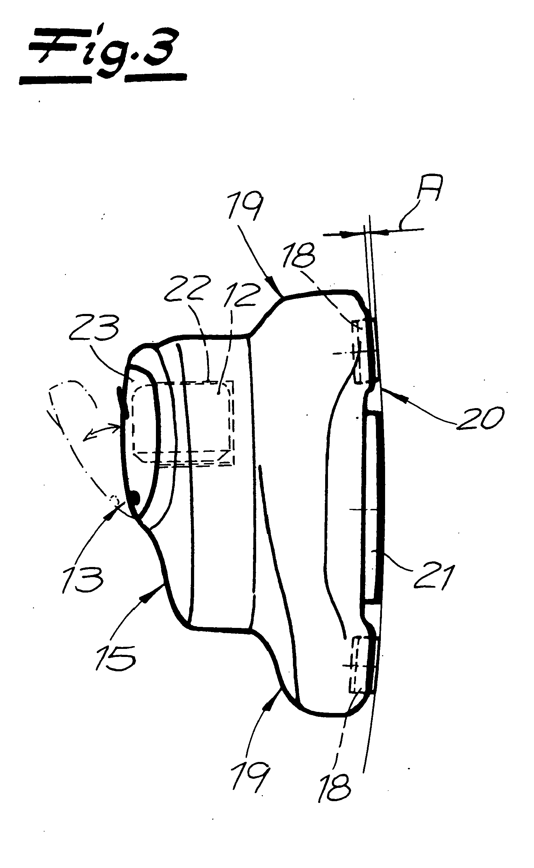

Zu den implantierbaren bzw. implantierten Befestigungselementen 17 korrespondieren Gegenbefestigungselemente 18, mit denen die Signaleinheit 11, 11', 12, 13, 14, 14', 14", 15 ausgerüstet ist. Tatsächlich sind die Gegenbefestigungselemente 18 im Innern des Signalgehäuses 15 untergebracht und finden sich ausweislich der Fig. 3 jeweils in beidendseitigen Auslegern 19 des kapselartigen und abgerundet quaderförmigen Signalgehäuses 15.

Bei dem jeweiligen Befestigungselement 17 und dem zugehörigen Gegenbefestigungselement 18 handelt es sich um zwei Magnete 17, 18 oder auch einen Magneten 17 und ein magnetisierbares Element 18, wie beispielsweise eine Stahlscheibe oder Eisenscheibe. Jedenfalls wird die externe Signaleinheit 11, 11', 12, 13, 14, 14', 14", 15 mit Hilfe ihres Gegenbefestigungselementes 18 bzw. ihrer beiden Gegenbefestigungselemente 18 lösbar an dem implantierten bzw. implantierbaren Befestigungselement 17 bzw. den beiden Befestigungselementen 17 lösbar festgelegt.

Dadurch kann die Signaleinheit 11, 11', 12, 13, 14, 14', 14", 15 problemlos beispielsweise zum Austausch der Energieversorgungseinrichtung respektive Batterie 12 von der fraglichen Anbringungsstelle an einer Anlagefläche 20 am Kopf des Bedieners entfernt werden. Außerdem ermöglicht diese lösbare Befestigung den Austausch und/oder die Aktualisierung des Signalprozessors 14 und/oder der Signalbearbeitungs- und Verstärkungseinheit 14', 14" mit neuer und verbesserter Software. Dabei reichen die von den Magneten bzw. dem jeweiligen Magnet 17, 18 und dem magnetisierbaren Element 17, 18 aufgebrachten Haftkräfte jeweils aus, das nur wenige Gramm schwere Signalgehäuse 15 am definierten Ort im Bereich der Anlagefläche 20 zu halten. Tatsächlich kommen an dieser Stelle scheibenartige Magnete mit kreisförmigem Durchmesser zum Einsatz, die einen Radius von nur wenigen Millimetern und eine Stärke ebenfalls im Millimeterbereich aufweisen, folglich problemlos implantiert werden können. Dennoch werden Haftkräfte im Bereich von > 0,1 N aufgebracht, die die Gewichtskräfte von < 0,1 N deutlich überschreiten.

Man erkennt, dass im Rahmen des Ausführungsbeispiels jeweils zwei Paare Befestigungselement 17/Gegenbefestigungselement 18 vorgesehen sind, die sich jeweils randseitig des Signalgehäuses 15 finden. Anhand der Fig. 3 wird deutlich, dass die in das Signalgehäuse 15 eingebauten Gegenbefestigungselemente 18 verstellbar ausgebildet sind. Grundsätzlich können auch die Befestigungselemente 17 verstellt werden, was jedoch nicht gezeigt ist. Durch die Höhenverstellbarkeit der Gegenbefestigungselemente 18 lässt sich ihr Abstand A zu den Befestigungselementen 17 verändern und folglich auch der Abstand der Grundplatte 21 des Signalgehäuses 15 von der Anlagefläche 20 am Kopf des Bedieners.

Die fragliche Grundplatte 21 ist austauschbar ausgebildet und kann als Abdruckplatte ausgeführt sein. Auf diese Weise lässt sich beim Bediener ein Abdruck von der Anlagefläche 20 nehmen und folglich die Grundplatte 21 an die Kontur der Anlagefläche 20 individuell anpassen, um den Tragekomfort zu erhöhen. Hierzu trägt auch die Möglichkeit bei, gleichsam den Anpressdruck des Signalgehäuses 15 an die fragliche Anlagefläche 20 dadurch zu variieren, dass die Gegenbefestigungselemente 18 entsprechend in ihrer Höhe eingestellt werden. Zu diesem Zweck sind die Gegenbefestigungselemente 18 mit einem angedeuteten Gewindezapfen ausgerüstet, der in einen Gewindegang im Signalgehäuse 15 eintaucht, um den fraglichen Abstand A verändern zu können.

Das Signalgehäuse 15 weist eine mit Hilfe eines Deckels 23 verschließbare Öffnung respektive Ausnehmung 22 auf, die zur Aufnahme der auswechselbaren Energieversorgungseinheit 12 ausgerüstet ist. Schließlich lässt sich die Sendespulenanordnung 11 - wie in Fig. 2 angedeutet - gegenüber dem Signalgehäuse 15 und folglich auch der Kopfhaut 16 des Bedieners verschieben. Dadurch wird etwaigen Unregelmäßigkeiten der Anlagefläche 20 Rechnung getragen und lässt sich eine optimale Anlage des Signalgehäuses 15 an der Anlagefläche 20 ebenso erreichen, wie eine einwandfreie drahtlose Kopplung von einerseits der Sendespulenanordnung 11 und andererseits der Empfangsspulenanordnung 5. Das Gleiche gilt für den elektromechanischen Wandler 11' nach Fig. 1, der ebenfalls verschiebbar gegenüber der Kopfhaut 16 und folglich dem Gehäuse 15 in diesem gelagert ist.

## Patentansprüche

1. Hörgerät, mit zumindest einer externen Signaleinheit (11, 11', 12, 13, 14', 14", 15), wobei wenigstens ein implantierbares Befestigungselement (17) für die mit einem Gegenbefestigungselement ausgerüstete Signaleinheit (11, 11', 12, 13, 14', 14", 15) vorgesehen ist,
**dadurch gekennzeichnet,dass**
ein die Signaleinheit (11, 11', 12, 13, 14', 14", 15) aufnehmendes Gehäuse (15) aus einer die Signaleinheit (11, 11', 12, 13, 14', 14", 15) umschließenden aushärtenden Kunststoffmasse gefertigt ist.

2. Hörgerät nach Anspruch 1, **dadurch gekennzeichnet, dass** das Befestigungselement (17) und das Gegenbefestigungselement (18) als zwei Magnete (17, 18) oder ein Magnet (17) und ein magnetisierbares Element (18) ausgebildet sind.

3. Hörgerät nach Anspruch 1 oder 2, **dadurch gekennzeichnet, dass** zwei oder mehr Paare Befestigungselement (17)/Gegenbefestigungselement (18) vorgesehen sind, beispielsweise jeweils randseitig des Gehäuses (15) der Signaleinheit (11, 11', 12, 13, 14, 14', 14", 15).

4. Hörgerät nach einem der Ansprüche 1 bis 3, **dadurch gekennzeichnet, dass** das Befestigungselement (17) und/oder das Gegenbefestigungselement (18) verstellbar, insbesondere höhenverstellbar, ausgebildet ist, um den Abstand (A) einer Grundplatte (21) des Gehäuses (15) von einer Anlagefläche (20) am Kopf eines Bedieners zu verändern.

5. Hörgerät nach Anspruch 4, **dadurch gekennzeichnet, dass** die Grundplatte (21) austauschbar ausgebildet und als Abdruckplatte ausgeführt ist.

6. Hörgerät nach einem der Ansprüche 1 bis 5, **dadurch gekennzeichnet, dass** die Signaleinheit (11, 11', 12, 13, 14, 14', 14", 15) wenigstens ein Mikrophon (13), einen Signalprozessor (14) und/oder eine Signalbearbeitungs- und Verstärkungseinheit (14', 14") sowie eine Sendespulenanordnung (11) und/oder einen elektromechanischen Wandler (11') aufweist.

7. Hörgerät nach Anspruch 6, **dadurch gekennzeichnet, dass** die Sendespulenanordnung (11) und/oder der elektromechanische Wandler (11') verschiebbar gegenüber dem Gehäuse (15) ausgebildet ist.

8. Hörgerät nach einem der Ansprüche 1 bis 7, **dadurch gekennzeichnet, dass** die Signaleinheit (11, 11', 12, 13, 14, 14', 14", 15) am Mastoid angeordnet ist.

9. Hörgerät nach einem der Ansprüche 1 bis 8, **dadurch gekennzeichnet, dass** das Gehäuse (15) als kapselartiges Kunststoffgehäuse ausgebildet ist, welches lediglich eine verschließbare Öffnung (22) zur Aufnahme einer Energieversorgungseinheit (12) aufweist.
